# EUROPEAN PATENT APPLICATION

(11) **EP 1 510 209 A1**
(43) Date of publication of application: **02.03.2005**
(21) Application number: 03723187.5
(22) Date of filing: 24.04.2003
(51) Int. Cl.: A61K 31/166, A61P 1/00, A61P 1/04, A61P 43/00

(54) **THERAPEUTIC AGENT FOR INFLAMMATORY INTESTINAL DISEASES CONTAINING HYDROXAMIC ACID COMPOUND AS ACTIVE INGREDIENT**

(30) Priority: 25.04.2002 JP 2002124789
(71) Applicant: ONO PHARMACEUTICAL CO., LTD., Osaka-shi, Osaka 541-8526 (JP)
(72) Inventor: MAEDA, Yoshizo; c/o Pharmaceutical Co., Ltd, Mishima-gun, Osaka 618-8585 (JP); YAMASHITA, Kazunori; c/o Pharmaceutical Co., Ltd, Mishima-gun, Osaka 618-8585 (JP); KOYANAGI, Takashi; c/o Pharmaceutical Co., Ltd, Mishima-gun, Osaka 618-8585 (JP)
(74) Representative: Henkel, Feiler & Hänzel
(86) International application number: PCT/JP2003/005251
(87) International publication number: WO 2003/090735

(57) **Abstract**

A treatment and/or prevention agent for inflammatory bowel disease comprising a hydroxamic acid compound of formula (I) 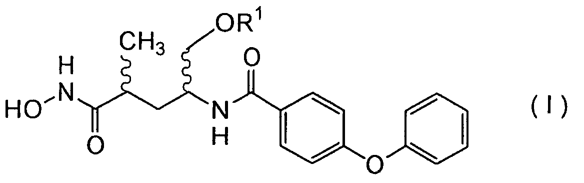 wherein R¹ is hydrogen, C1-8 alkyl or C1-8 alkyl substituted by -OR², R² is hydrogen, C1-8 alkyl, benzyl, or C1-8 alkyl substituted by C1-8 alkoxy; or a pharmaceutical acceptable salt thereof as active ingredient.

## Description

### Technical Field

This invention relates to a treatment and/or prevention agent for inflammatory bowel disease. More particularly, this invention relates to a treatment and/or prevention agent for inflammatory bowel disease comprising a hydroxamic acid compound of formula (I): 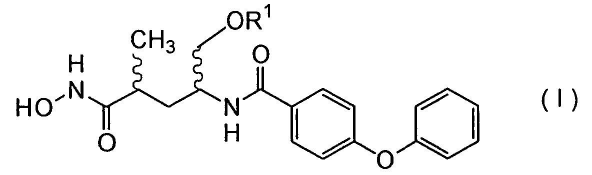 wherein all symbols are as hereinafter, or a pharmaceutical acceptable salt thereof as active ingredient.

### Background

Inflammatory bowel disease is chronic inflammatory disease, which is associated with erosion or ulcer on intestinal lining. The cause is thought variously, but not be ascertained. Therefore, there are no radical treatments presently, so palliative treatments are designed. For example, Salazosulfapyridine, Mesalazine, and adrenocortical steroid were used mainly as a suppressant of bowel inflammation. However, the above treatments will cause recurrence of an inflammation frequently, and cause some side-effects, such as a hyper-reactive symptom, a digestive system symptom, nausea, headache, moon face, acne, insomnia, so they are not fully satisfied.

In the specification of WO 99/19296, aminobutyric acid derivatives of formula (Z): 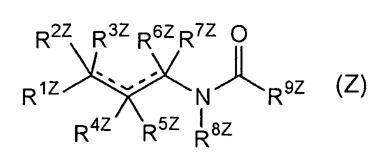 wherein R^{1Z} is -COOR^{10Z}, -CONHOR^{10Z}, etc.; R^{10Z} is hydrogen, C1-8 alkyl, etc.; R^{2Z}, R^{3Z}, R^{4Z}, R^{5Z}, R^{6Z}, and R^{7Z} each, in dependently, is (1) hydrogen, (2) C1-8 alkyl, (3) C2-8 alkenyl, (4) -OR^{11Z}, etc.; when R^{8Z} is (1) hydrogen, (2) C1-8 alkyl, then R^{9Z} is 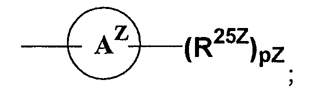 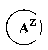 is a carbocyclic ring or a heterocyclic ring;
R^{25Z} is -E^{Z}-G^{Z} ; E^{Z} is (1) a single bond, (2) -CONR^{33Z}, (3) -NR^{33Z}CO-, (4) -CO-O-, etc.; G^{Z} is (1) hydrogen, (2) C1-8 alkyl, (3) Cyc4^{Z}, etc.;
having a metalloproteinase inhibitory activity, was reported. And the compound was disclosed to be useful for a treatment and/or prevention of rheumatoid disease, arthrosteitis, unusual bone resorption, osteoporosis, periodontitis, interstitial nephritis, arteriosclerosis, pulmonary emphysema, cirrhosis, cornea injury, metastasis of, invasion of or growth of tumor cells, autoimmune diseases (e.g. Crohn's disease, Sjogren's syndrome), diseases caused by vascular emigration or infiltration of leukocytes, arterialization, multiple sclerosis, aorta aneurysm, endometriosis.

On the other hand, medically speaking, causes of inflammatory bowel disease, especially causes of ulcerative colitis are wide variety, and so the treatments are also varied. There are no description and suggestion in the above specification that a compound of formula (I) is useful for a treatment and/or prevention of inflammatory bowel disease, especially ulcerative colitis, that is caused by complex and unconfirmed factors.

Moreover, a treatment for inflammatory bowel disease by metalloproteinase inhibitors is not generally confirmed method.

### Disclosure of the Invention

As previously noted, under it was eagerly looking forward to supply a treatment and/or prevention agent for inflammatory bowel disease, energetic investigations have been carried out in order to accomplish the above purpose by the present inventors. They have found first that a hydroxamic acid compound of formula (I) or a pharmaceutical acceptable salt thereof is useful as a treatment and/or prevention agent for inflammatory bowel disease, and accomplished the present invention.

This invention relates to a treatment and/or prevention agent for inflammatory bowel disease comprising a hydroxamic acid compound of formula (I): 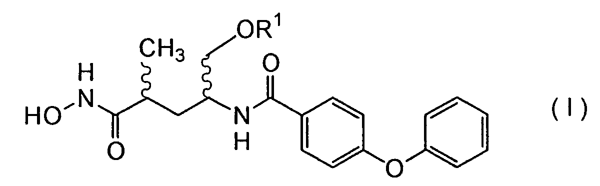 wherein R¹ is hydrogen, C1-8 alkyl or C1-8 alkyl substituted by -OR², R² is hydrogen, C1-8 alkyl, benzyl, or C1-8 alkyl substituted by C1-8 alkoxy, or a pharmaceutical acceptable salt thereof as active ingredient.

### Detailed Description

In the present invention, C1-8 alkyl is straight-chain or branched-chain C1-8 alkyl selected from methyl, ethyl, propyl, isopropyl, butyl, isobutyl, sec-butyl, tert-butyl, pentyl, isopentyl, neopentyl, hexyl, isohexyl, heptyl and octyl.

In the present invention, C1-8 alkyl substituted by -OR² is straight-chain or branched-chain C1-8 alkyl selected from methyl, ethyl, propyl, isopropyl, butyl, isobutyl, sec-butyl, tert-butyl, pentyl, isopentyl, neopentyl, hexyl, isohexyl, heptyl and octyl, which substituted by one of -OR².

In the present invention, C1-8 alkyl substituted by C1-8 alkoxy is straight-chain or branched-chain C1-8 alkyl selected from methyl, ethyl, propyl, isopropyl, butyl, isobutyl, sec-butyl, tert-butyl, pentyl, isopentyl, neopentyl, hexyl, isohexyl, heptyl and octyl, which substituted by one of straight-chain or branched-chain C1-8 alkoxy selected from methoxy, ethoxy, propoxy, isopropoxy, butoxy, isobutoxy, sec-butoxy, tert-butoxy, pentyloxy, isopentyloxy, neopentyloxy, hexyloxy, isohexyloxy, heptyloxy and octyloxy.

In the present invention, 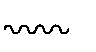 shows that the bond is an isomer resulting from the presence of an asymmetric carbon or a mixture thereof. Concretely, it shows the bond is in front of paper, that is, α-configuration 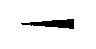 , the bond is on the other side of paper, that is, β-configuration 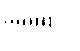 or a mixture thereof.

Unless otherwise specified, all isomers are included in the present invention. For example, alkyl and alkoxy include straight and branched isomers. Isomers resulting from the presence of asymmetric carbon(s) (R-configuration, S-configuration, α-configuration, β-configuration, enantiomers, diastereoisomers), optically active compounds having optical rotation (D, L, d, 1-configuration), polar compounds obtained by chromatographic separations (highly polar compound, less polar compound), equilibrium compounds, the mixtures are existed by free ratio, racemic mixtures are included in the present invention.

In the present invention, all groups represented by R¹ are preferable. More preferable group is hydrogen, C1-4 alkyl or C1-4 alkyl substituted by one of -OR², and preferable R² is hydrogen, C1-4 alkyl, benzyl, or C1-4 alkyl substituted by one of C1-4 alkoxy. Especially preferable R¹ is hydrogen or C1-4 alkyl substituted by one of -O-(C1-4 alky). C1-4 alkyl is methyl, ethyl, propyl, isopropyl, butyl, isobutyl, sec-butyl or tert-butyl. C1-4 alkoxy is methoxy, ethoxy, propoxy, isopropoxy, butoxy, isobutoxy, sec-butoxy or tert-butoxy. Especially methyl and ethyl are preferable.

In the compound of formula (I) of the present invention, the compound of formula (I'): 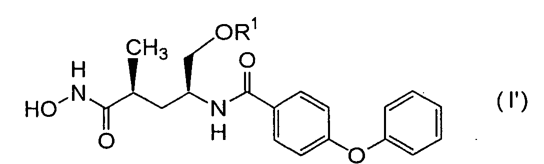 wherein all symbols are as hereinbefore, is preferable.

In the present invention, concretely compounds are N-hydroxy-5-hydroxy-2(S)-methyl-4(S)-(4-phenoxybenzoyl)aminopentanamide or a pharmaceutical acceptable salt thereof, and N-hydroxy-5-ethoxymethyloxy-2(S)-methyl-4(S)-(4-phenoxybenzoyl)aminopentanamide or a pharmaceutical acceptable salt thereof.

The compound of the present invention may be used a corresponding pharmaceutically acceptable salt. Non-toxic and water-soluble salts are preferable. Suitable salts, for example, include: salts of alkali metals (e.g. potassium, sodium), salts of alkaline earth metals (e.g. calcium, magnesium), ammonium salts, salts of pharmaceutically acceptable organic amines (e.g. tetramethylammonium, triethylamine, methylamine, dimethylamine, cyclopentylamine, benzylamine, phenethylamine, piperidine, monoethanolamine, diethanolamine, tris(hydroxymethyl)amine, lysine, arginine, N-methyl-D-glucamine). The compounds of the present invention or a pharmaceutical acceptable salt thereof may be converted into the corresponding hydrates by conventional means.

The compound of formula (I) is prepared by, for example, a method described in the specification of WO 99/19296.

### [Toxicity]

The toxicity of the compounds of the present invention is very low and therefore, the compounds may be considered safe for pharmaceutical use. For example, a minimum of lethal dose of the compound (2) by single oral administration to rat was 2000 mg/kg.

### Industrial Applicability

### [Application for Pharmaceuticals]

The hydroxamic acid compound or a pharmaceutical acceptable salt thereof of the present invention, is useful for a treatment and/or prevention of inflammatory bowel disease, especially ulcerative colitis, in animals including human beings, especially human beings.

The compound of formula (I) or a pharmaceutical acceptable salt thereof may be administered in combination with other medicaments for the purpose of
1) complement and/or enhancement of preventing and/or treating effect,
2) improvement of dynamics and absorption of the compound, and lowering of dose, and/or
3) alleviation of side effect of the compound.

The compound of formula (I) may be administered in combination with other medicaments as a composition in one drug product comprising these components, or may be administered separately. When they are administered independently, they may be administered simultaneously or with time lag. Administering with time lag includes the method of administering the compound of formula (I) before other medicaments and vice versa, and they may be administered in the same route or not.

The above combination takes effects on whichever disease treating and/or preventing effect of the compound of formula (I) is complemented and/or enhanced.

As other medicaments to complement and/or to enhance the preventing and/or treating effect of the compound of formula (I) for inflammatory bowel disease, for example, prostaglandin synthetase inhibitor, steroid, immunosuppressant, leukotriene receptor antagonist, TNFα antagonist, adhesion molecules inhibitor, antiulcer drug of the gastrointestinal tract, anticholinergic agent, antibiotics, 5-lipoxygenase inhibitor, phosphodiesterase inhibitor, elastase inhibitor etc. are given.

As prostaglandin synthetase inhibitor, for example, Salazosulfapyridine, Mesalazine, Olsalazine, 4-Aminosalicylic acid, JTE-522, Auranofin, Carprofen, Diphenpyramide, Flunoxaprofen, Flurbiprofen, Indometacin, Ketoprofen, lornoxicam, Loxoprofen, Meloxicam, Oxaprozin, Parsalmide, Piproxen, Piroxicam, Piroxicam betadex, Piroxicam cinnamate, Tropineindometacinate, Zaltoprofen, Pranoprofen are given.

As steroid of internal medicine and injection, for example, Cortisone acetate, Hydrocortisone, Hydrocortisone sodium phosphate, Hydrocortisone sodium succinate, Fludrocortisone acetate, Prednisolone, Prednisolone acetate, Prednisolone sodium succinate, Prednisolone butylacetate, Prednisolone sodium phosphate, Halopredone acetate, Methylprednisolone, Methylprednisolone acetate, Methylprednisolone sodium succinate, Triamcinolone, Triamcinolone acetate, Triamcinolone acetonide, Dexamethasone, Dexamethasone acetate, Dexamethasone sodium phosphate, Dexamethasone palmitate, Paramethason acetate, Betamethasone are given.

As immunosuppressant, for example, Protopic (FK-506), Methotrexate, Ciclosporin, Ascomycin, Leflunomide, Bucillamine, Salazosulfapyridine are given.

As leukotriene receptor antagonist, for example, Pranlukast hydrate, Montelukast, Zafirlukast, Seratrodast, MCC-847, KCA-757, CS-615, YM-158, L-740515, CP-195494, LM-1484, RS-635, A-93178, S-36496, BIIL-284, ONO-4057 are given.

As anticholinergic agent, for example, Mepenzolate bromide, Ipratropium bromide are given.

As phosphodiesterase inhibitor, for example, Rolipram, Cilomilast (Ariflo ™), Bay-19-8004, NIK-616, Roflumilast (BY-217), Cipamfylline (BRL-61063), Atizoram (CP-80633), SCH-351591, YM-976, V-11294A, PD-168787, D-4396, IC-485 that are phosphodiesterase 4 inhibitor, are given.

As elastase inhibitor, for example, ONO-5046, ONO-6818, MR-889, PBI-1101, EPI-HNE-4, R-665, ZD-0892, ZD-8321, GW-311616, AE-3763 are given.

Weight ratio of the compound of formula (I) and other medicaments is not limited.

A combination of any two or more of other medicaments may be administered.

In other medicaments to complement and/or to enhance the preventing and/or treating effect of the compound of formula (I), medicaments that not only exist now but also may be found in the future on the basis of above mechanisms are included.

For the purpose described above, the compound of formula (I) of the present invention or a concomitant drug combined the compound of formula (I) with other medicaments may be normally administered systemically or topically, usually by oral or parenteral administration.

The doses to be administered are determined depending upon, for example, age, body weight, symptom, the desired therapeutic effect, the route of administration, and the duration of the treatment, etc. In the human adult, the doses per person at a time are generally from 1 mg to 1000 mg, by oral administration, up to several times per day, and from 1 mg to 100 mg, by parenteral administration (preferably intravenous administration), up to several times per day, or continuous administration between 1 and 24 hours per day into vein.

As mentioned above, the doses to be used depend upon various conditions. Therefore, there are cases wherein doses lower than or greater than the ranges specified above may be used.

A preferable amount by oral administration is an amount selected from (i) 12.5 mg/ one solid composition, (ii) 25 mg/ one solid composition, (iii) 50 mg/ one solid composition and (iv) 100 mg/ one solid composition, or an amount by 1-3 times administration per one day and 1-4 tablets per one administration, which are selected and combined from the above solid compositions, that is 1-12 tablets per one day, for the underlying a condition of target disease.

The compound of formula (I) or concomitant drug combined the compound of formula (I) with other medicaments may be administered for oral or parenteral administration.

Composition for oral administration is, for example, solid compositions, liquid compositions or the other compositions.

Solid compositions for oral administration include compressed tablets, pills, capsules, dispersible powders and granules. Capsules include hard capsules and soft capsules. Compressed tablets are preferable.

In such solid compositions, one or more of the active compounds may be admixed with vehicles such as lactose, mannitol, glucose, hydroxypropyl cellulose, microcrystalline cellulose, starch, polyvinylpyrrolidone or magnesium aluminometasilicate. The compositions may comprise, in accordance with the conventional process, additives other than the inert diluent, for example, lubricants such as magnesium stearate; disintegrating agents such as cellulose calcium glycolate; stabilizer such as lactose; and solubilizing agent such as glutamic acid or aspartic acid. Tablets or pills may be coated with a film of a gastric soluble or enteric substance such as sucrose, gelatin, hydroxypropyl cellulose or hydroxypropyl methylcellulose phthalate, or with two or more layers, if necessary. Furthermore, capsules made of a substance which can be absorbed in the body, for example, gelatin, are included.

Liquid compositions for oral administration include pharmaceutically acceptable suspensions and emulsions, solutions, syrups and elixirs. In such compositions, one or more of the active compounds may be dissolved, suspended or emulsified into diluents commonly used in the art (such as purified water, ethanol). Besides such liquid compositions may also comprise some additives, such as wetting agents, suspending agents, sweetening agents, flavoring agents, aroma or preservative.

The other compositions for oral administration include sprays which comprise one or more active compounds and are formulated in a manner known per se in the art. The compositions may comprise, in addition to an inert diluent, a stabilizer such as sodium bisulfite and a tonicity agent such as sodium chloride, sodium citrate or citric acid. The preparation process of sprays is described in detail in, for example, U.S. Patent Nos. 2,868,691 and 3,095,355.

Composition for parenteral administration is, for example, injections, liniments or suppositories, ophthalmic solution, respiratory tonic etc.

In the present invention, injections for parenteral administration include sterile aqueous and/or non-aqueous solutions, suspensions and emulsions. The aqueous solutions or suspensions include, for example, distilled water for injection and saline. The non-aqueous solutions or suspensions include propylene glycol, polyethylene glycol, vegetable oils such as olive oil, alcohol such as ethanol and Polysorbate 80 (trade mark). Furthermore, sterile aqueous and non-aqueous solutions, suspensions, and emulsions may be used in combination. Such compositions may additionally comprise adjuvants such as antiseptic, humectant, emulsifier, dispersant, stabilizer, such as lactose; and solubilizing agent, such as glutamic acid and aspartic acid. They are sterilized by filtration through a bacteria retaining filter, the addition of a fungicide, or irradiation. Also, a sterile solid composition is prepared and then, for example, a freeze-dried product may be dissolved in sterilized or sterile distilled water for injection or another sterile solvent before use.

The other compositions for parenteral administration include liquids for external use, liquid for external use, ointments, endermic liniments, suppositories for intrarectal administration which comprise one or more of the active substances and may be prepared by methods known per se.

### Brief Description of Drawings

Figure 1 shows a measurement result of the injured area by an administration of 0.3 and 3 mg/kg/day of the compound (1).
Figure 2 shows a measurement result of the wet weight by an administration of 0.3 and 3 mg/kg/day of the compound (1).
Figure 3 shows a measurement result of the length of all colons by an administration of 1, 3 and 10 mg/kg/day of the compound (2).
Figure 4 shows a measurement result of the wet weight by an administration of 1, 3 and 10 mg/kg/day of the compound (2).

### Best Mode for Carrying out the Invention

The effect of the compound of the present invention on inflammatory bowel disease was confirmed by, for example, following experiments, but not to limit the present invention.

### Experiment 1: Acetic acid-induced colitis model

### [Method]

Male SD (CD) IGS rats (7 weeks old) were anesthetized with sodium pentobarbital. Colitis was induced by intracolonic instillation of 0.25 mL of 5% acetic acid with a disposable sonde for oral administration for mouse, which is attached to 1mL syringe (Terumo). The sonde was inserted to 5 cm from anus and acetic acid was infused for 10 seconds. After the removal of the sonde, the anus was plugged for 1 minute. Then the colon was washed with approximate 10 mL of physiological saline with a disposable sonde for oral administration for rat, which was attached to 50 mL syringe (Terumo), and the sonde was inserted to 8 cm from anus.

The test compound or vehicle was administered to animals before 30 minutes and after 8 hours of the colitis induction, at each time half of a dairy dose was administered.

After 24 hours of colitis induction, all colons which from anus to cecum, were removed, the content was washed away with saline solution. After trimming of the washed colon, the colon was cut at 9 cm from anus, wiped out the solution, and weighed with electronic measure. Then the injured area (mm²) of dissected colon was measured by image analysis.

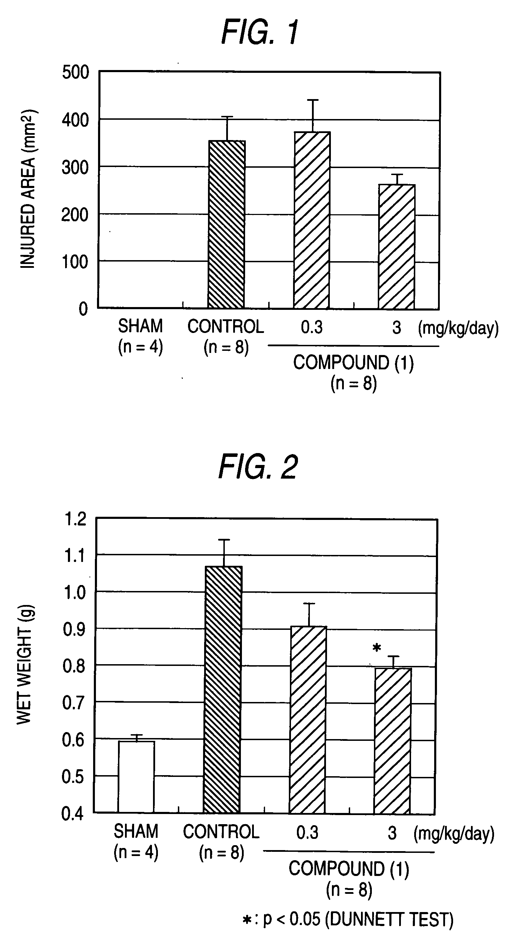

N-hydroxy-5-hydroxy-2(S)-methyl-4(S)-(4-phenoxybenzoyl)aminopentanamide (compound (1)) was used as a test compound. Compound (1) was administered to animals at doses of 0.3 or 3mg/kg/day. Figure 1 and Figure 2 show the effect of compound (1) on the injured area and the wet weight.

These results indicated that compound (1) suppressed the increase in intestine edema, and has suppressive tendency to the injured area.

### Experiment 2: TNBS-induced colitis model

### [Methods]

Male SD (CD) IGS rats (7 weeks old) were anesthetized with sodium pentobarbital. A flexible sonde for oral administration was inserted to 8 cm from the anus. Colitis was induced by intracolonic instillation of 50 mg of 2,4,6-trinitrobenzenesulfonic acid (TNBS) dissolved in 0.25 mL of 20% ethanol, and the anus was plugged and leaved for 2 hours. Test compound was administered at 30 minutes before and 8 hours after the TNBS induction, since the next day of TNBS induction they were administered half of a dairy dose at each time, twice daily in the morning and the evening. Three days later the animals were sacrificed with bleeding under ether anesthetization. All colons were removed, the contents was washed out with saline solution, and then measured the length. After the trimming of the colons, 9 cm segment from the anus was obtained, the water was removed on the paper, and the wet weight was measured with electric scale.

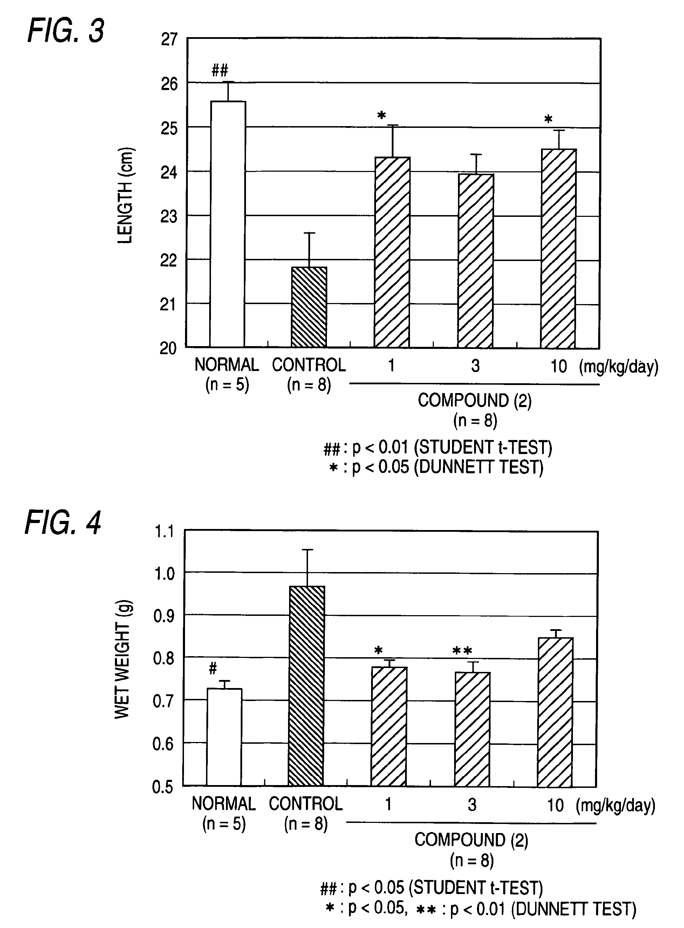

N-hydroxy-5-ethoxymethyloxy-2(S)-methyl-4(S)-(4-phenoxylbenzoil)aminopentanamide (compound (2)) was used as a test compound. Figure 3 and Figure 4 show the effects of compound (2) at the doses of 1, 3 and 10 mg/kg/day on the length and the wet weight of colons respectively.

These results indicated that compound (2) suppressed the shrinkage of colon and the increase in intestine edema.

In the above animal models, the compound of formula (I) showed the inhibitory effect on the injured area, the increase in intestine edema and the shrinkage of colon. Accordingly, it is evaluated that the compound of formula (I) is useful for a treatment of inflammatory bowel disease.

### Formulation example 1

The following components were admixed in conventional method and punched out to obtain 100 tablets each containing 50 mg of active ingredient.
· N-hydroxy-5-ethoxymethyloxy-2(S)-methyl-4(S)-(4-phenoxybenzoyl) aminopentanamide 5.0 g
· Carboxymethylcellulose calcium (disintegrating agent) 0.2 g
· Magnesium stearate (lubricating agent) 0.1 g
· Microcrystalline cellulose 4.7 g

### Formulation example 2

The following components were admixed in conventional method. The solution was sterilized in conventional manner, placed 5 ml portions into ampoules and freeze-dried to obtain 100 ampoules each containing 20 mg of the active ingredient.
· N-hydroxy-5-ethoxymethyloxy-2(S)-methyl-4(S)-(4-phenoxybenzoyl) aminopentanamide 2.0 g
· Mannitol 20 g
· Distilled water 500 ml

## Claims

1. An agent for treatment and/or prevention of inflammatory bowel disease comprising a hydroxamic acid compound of formula (I): 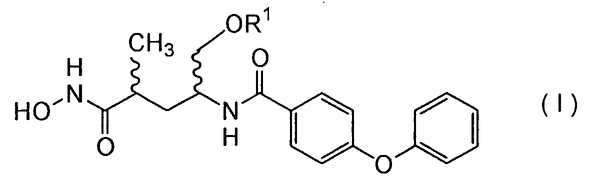 wherein R¹ represents hydrogen, C1-8 alkyl or C1-8 alkyl substituted by -OR²; R² represents hydrogen, C1-8 alkyl, benzyl, or C1-8 alkyl substituted by C1-8 alkoxy, or a pharmaceutical acceptable salt thereof as an active ingredient.

2. The agent for treatment and/or prevention according to claim 1, wherein the inflammatory bowel disease is ulcerative colitis.

3. The agent for treatment and/or prevention according to claim 1, wherein the compound is
(1) N-hydroxy-5-hyclroxy-2(S)-methyl-4(S)-(4-phenoxybenzoyl)aminopentanamide or a pharmaceutical acceptable salt thereof, or
(2) N-hydroxy-5-ethoxymethyloxy-2(S)-methyl-4(S)-(4-phenoxybenzoyl)aminopentanamide or a pharmaceutical acceptable salt thereof.

4. The agent for treatment and/or prevention according to claim 3, which contains an amount of 12.5 mg to 100 mg of N-hydroxy-5-hydroxy-2(S)-methyl-4(S)-(4-phenoxybenzoyl)aminopentanamide or a pharmaceutical acceptable salt thereof per one solid preparation.

5. The agent for treatment and/or prevention according to claim 3, which contains an amount of 12.5 mg to 100 mg of N-hydroxy-5-ethoxymethyloxy-2(S)-methyl-4(S)-(4-phenoxybenzoyl)aminopentanamide or a pharmaceutical acceptable salt thereof per one solid preparation.

6. Use of a hydroxamic acid compound of formula (I): 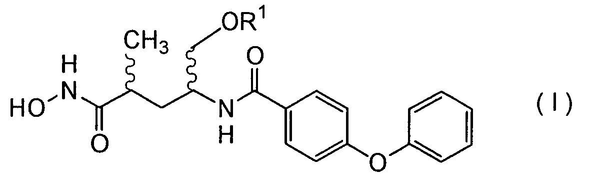 wherein R¹ represents hydrogen, C1-8 alkyl or C1-8 alkyl substituted by -OR²; R² represents hydrogen, C1-8 alkyl, benzyl, or C1-8 alkyl substituted by C1-8 alkoxy, or a pharmaceutical acceptable salt thereof for the manufacture of a medicament for the treatment and/or prevention of inflammatory bowel disease.

7. The use according to claim 6, wherein the inflammatory bowel disease is ulcerative colitis.

8. The use according to claim 6, wherein the compound is
(1) N-hydroxy-5-hydroxy-2(S)-methyl-4(S)-(4-phenoxybenzoyl)aminopentanamide or a pharmaceutical acceptable salt thereof, or
(2) N-hydroxy-5-ethoxymethyloxy-2(S)-methyl-4(S)-(4-phenoxybenzoyl)aminopentanamide or a pharmaceutical acceptable salt thereof.

9. The use according to claim 8, wherein the amount of N-hydroxy-5-hydroxy -2(S)-methyl-4(S)-(4-phenoxybenzoyl)aminopentanamide or a pharmaceutical acceptable salt thereof in one solid preparation is 12.5 mg to 100 mg.

10. The use according to claim 8, wherein the amount of N-hydroxy-5-ethoxymethyloxy -2(S)-methyl-4(S)-(4-phenoxybenzoyl)aminopentanamide or a pharmaceutical acceptable salt thereof in one solid preparation is 12.5 mg to 100 mg.

11. A method for the treatment and/or prevention of inflammatory bowel disease in mammals, which comprises administering to a mammal an effective amount of a hydroxamic acid compound of formula (I): 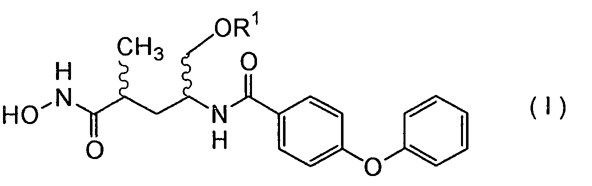 wherein R¹ represents hydrogen, C1-8 alkyl or C1-8 alkyl substituted by -OR²; R² represents hydrogen, C1-8 alkyl, benzyl, or C1-8 alkyl substituted by C1-8 alkoxy, or a pharmaceutical acceptable salt thereof.

12. The method for the treatment and/or prevention according to claim 11, wherein the inflammatory bowel disease is ulcerative colitis.

13. The method for the treatment and/or prevention according to claim 11, wherein the compound is
(1) N-hyd-roxy-5-hydroxy-2(S)-methyl-4(S)-(4-phenoxybenzoyl)aminopentanamide or a pharmaceutical acceptable salt thereof, or
(2) N-hydroxy-5-ethoxymethyloxy-2(S)-methyl-4(S)-(4-phenoxybenzoyl)aminopentanamide or a pharmaceutical acceptable salt thereof.

14. The method for the treatment and/or prevention according to claim 13, wherein the amount of N-hydroxy-5-hydroxy -2(S)-methyl-4(S)-(4-phenoxybenzoyl)aminopentanamide or a pharmaceutical acceptable salt thereof in one solid preparation is 12.5 mg to 100 mg.

15. The method for the treatment and/or prevention according to claim 13, wherein the amount of N-hydroxy-5-ethoxymethyloxy -2(S)-methyl-4(S)-(4-phenoxybenzoyl)aminopentanamide or a pharmaceutical acceptable salt thereof in one solid preparation is 12.5 mg to 100 mg.
